# EUROPEAN PATENT SPECIFICATION

(11) **EP 0 720 440 B1**
(45) Date of publication and mention of the grant of the patent: **05.11.2003**
(21) Application number: 94928175.2
(22) Date of filing: 20.09.1994
(51) Int. Cl.: A61B 5/00, A61B 10/00

(54) **MULTIPLE BIOPSY SAMPLING DEVICE**
VORRICHTUNG ZUR MEHRFACHEN PROBENENTNAHME
DISPOSITIF DE PRELEVEMENT DE PLUSIEURS ECHANTILLONS

(30) Priority: 20.09.1993 US 124272; 30.09.1993 US 129653; 29.10.1993 US 146447; 08.02.1994 US 193255; 08.02.1994 US 193298; 08.02.1994 US 193382
(43) Date of publication of application: 10.07.1996
(60) Divisional application: 03101894.8
(73) Proprietor: Boston Scientific Corporation, Natick, Massachusetts 01760 (US)
(72) Inventor: BANIK, Michael S., Cincinnati, OH 45241 (US); ROBINSON, Donald E., Hopkinton, MA 01748 (US)
(74) Representative: Brunner, Michael John
(86) International application number: US9410673
(87) International publication number: WO95008292

(56) References cited:
- WO-A-92/11882
- DE-A- 3 023 955
- DE-C- 935 625
- GB-A- 2 033 753
- US-A- 3 844 272
- US-A- 4 220 155
- US-A- 4 708 147
- US-A- 4 971 067
- US-A- 5 195 533
- US-A- 5 251 641

## Description

This invention relates to taking samples of tissue from the body.

Tissue samples can be examined in a laboratory to determine the presence of a pathological disorder (e.g. malignancy). Often, the samples must be obtained from deep within the body using a medical sampling instrument. It is usually best to obtain several samples around the location where the disorder is suspected so that the presence and progress of disease, if any, can be accurately determined. The samples must be catalogued according to the location from which each sample is taken and the integrity of the samples must be maintained for the subsequent laboratory analysis.

GB-A-2003753 discloses a medical instrument for removing a specimen form a patient's lung for analysis.

WO92/11882 discloses a safety needle which includes an elongate, tubular needle and a safety probe movable therein.

DE-A-935625 discloses an excision device to remove tissue samples from the human body having, at its distal end, a side-facing sampling assembly constructed to remove a tissue sample from the human body, wherein the side-facing sampling assembly has a cutting member near the distal end of the device which can be actuated in a first, rotational motion about the axis of the device to take a tissue sample and in a second, axial motion towards the proximal end of the device to remove the sample from the device.

US 4,220,155 discloses a surgical tool for use in removing the ovaries from large animals.

According to the present invention there is provided an instrument for obtaining tissue samples from sites deep within a body, the instrument having an elongate flexible proximal portion that is constructed to follow a long, tortuous path to said sites, and having, at its distal end, a side-facing sampling assembly constructed to remove a tissue sample from the body, including tissue specimens, polyps or the like, wherein said instrument is constructed to take multiple biopsy samples, without being withdrawn from the body, by means of a storage space defined along the axis of said instrument and proximal of the distal end of the instrument, suitable for storage of multiple, successively taken samples, and said side-facing sampling assembly having a cutting member near the distal end of said instrument actuatable in a first, rotational motion about the axis of said instrument to take a tissue sample from the body and in a second, limited axial motion towards the proximal end of the instrument to take said sample between a sample cutting position and a position in said storage space for disposing said sample axially into said storage space, said cutting member in use being returnable to the sample cutting position in preparation for taking a successive sample, the previous sample or samples being retained axially in said storage space, without withdrawing said cutting member or said instrument from the body.

Embodiments of this aspect may include one or more of the following features. The sampling element has an integral, axially adjacent sample-engaging surface for engaging a sample during axial motion of the element. The sampling assembly is a tube-form including a side-facing cutout defining a cutting surface and the sample engaging surface, and the storage space is defined by a portion of the tube-form adjacent the cutout. The instrument includes a retention formation constructed such that the sample can be moved in the proximal direction into contact with said retention formation by the second limited axial motion of the cutting member and said cutting member can be returned to the sample cutting position while said sample is retained axially stationary by said retention formation. The retention formation is a high friction surface. The sampling element is constructed to prevent abrasion of the sample against the retention formation as the sample is moved axially in the proximal direction. The instrument includes an outer sleeve wherein the cutting member is movable relative to the outer sleeve, and the retention formation is provided on the outer sleeve. The retention formation is provided on a small portion of the interior surface of the outer sleeve that is about equal to or less than the circumferential width and axial length of the side-facing cutout of the sampling assembly. The sampling assembly includes a scraping surface constructed to move between a tissue sample and a retention formation to facilitate transfer of the sample into the storage space as the cutting member returns to the sample cutting position. The retention formation is formed by sandblasting. The interior sample-contacting surfaces of the storage space include a low friction coating that allows easy axial relative displacement of the surfaces and the sample. The low friction coating is provided by a hydrogel coating. The instrument includes an axially movable sample-retaining element constructed to engage and maintain samples in the storage space as the cutting member moves axially into a cutting position.

Other features and advantages and methods of use follow.

We first briefly describe the drawings.
Fig. 1 is a perspective view of an embodiment of the invention being delivered into the body through an endoscope;
Fig. 2 is a cross-sectional view of an embodiment of the invention; Fig. 2a is a top view of the embodiment; Fig. 2b is a greatly enlarged view of the area in circle b in Fig. 2; and Fig. 2c is an end on cross section taken along the lines cc in Fig. 2;
Figs. 3-3e illustrate a use of the embodiment of Fig. 2;
Fig. 4 is a cross-sectional view of an additional embodiment of the invention; Fig. 4a is a top view of the additional embodiment;
Figs. 5-5f illustrate a use of the additional embodiment;
Fig. 6 is a cross-sectional view of an additional embodiment;
Fig. 6a is a cross-sectional view of an additional embodiment;
Fig. 7 is a cross-sectional view of another embodiment;
Fig. 8 is a cross-sectional view of another embodiment.

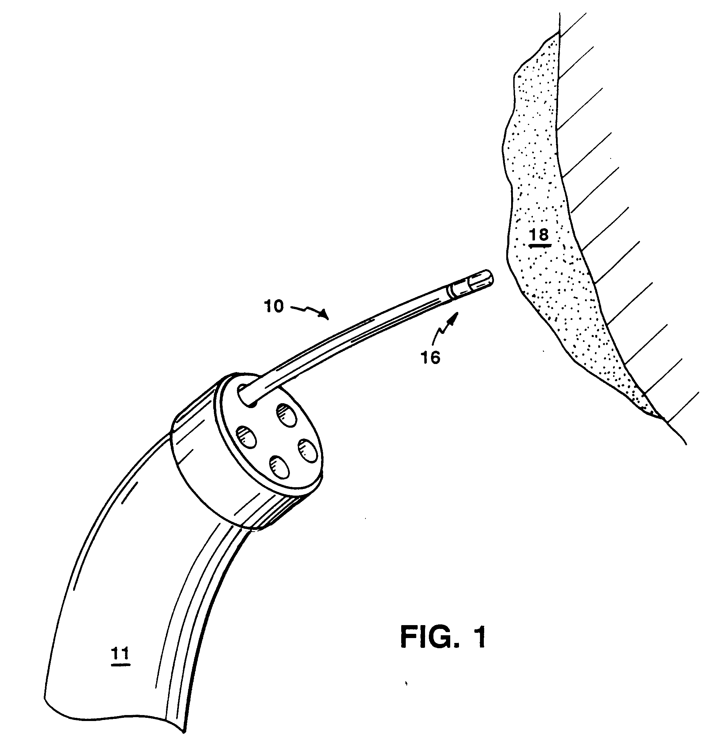

Referring to Fig. 1, the device 10 for multiple biopsy sampling may be delivered into the body through the channel of an endoscope device 11 (e.g., gastroscope, sigmoidoscope, or colonoscope). The endoscope device typically has a length of about 100-250 cm and a channel diameter of 2.0 - 3.8 mm, typically about 2.8 mm. A distal sampling portion 16 is extended from the endoscope for cutting and storing a sample of tissue from a body surface 18 of a patient (e.g. from a surface in the gastrointestinal tract or bronchial tract). The device has a diameter of preferably around 1.8 - 2.4 mm, typically about 2.3 mm or less and is of sufficient flexibility so it passes easily though the channel when the endoscope follows a tortuous body passageway. The endoscope may include other lumens for water, air, suction, and viewing, for example. Devices according to the invention can be adapted to be introduced to sites (e.g., urinary tract, reproductive organs, cardiac tissue, or the like) deep within the body by other means. For example, a device can be configured with a lumen so that it can be advanced over a guidewire, e.g., in vascular applications. The device may be passed through an introducer or guiding catheter in, e.g., cardiac applications. The sampling and storage arrangements may be useful in open surgery applications.

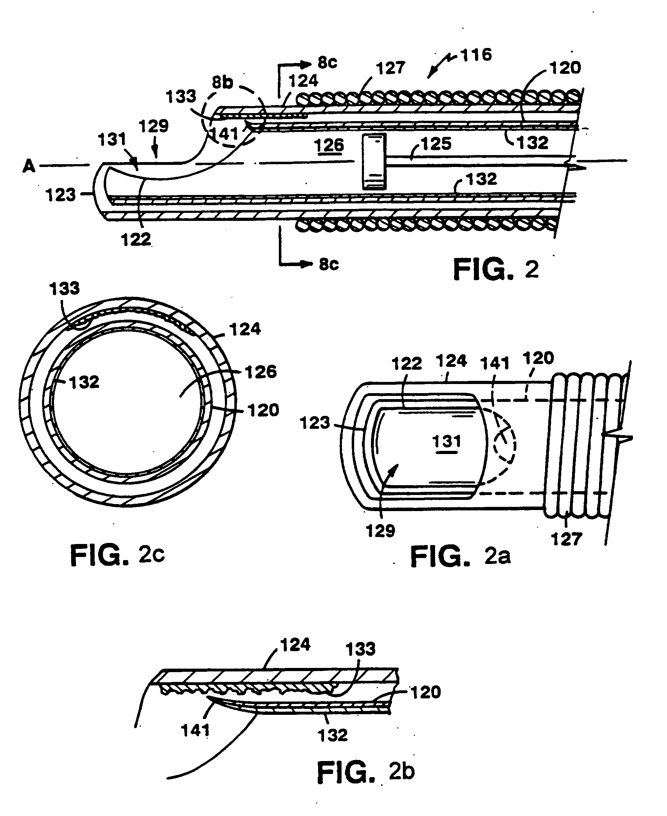

Referring to Figs. 2-2c, the sampling portion 116 includes an outer sleeve 124 concentrically positioned about an inner, generally tubular member 120. The sleeve 124 includes a side-facing opening 129 at its distal portion and a generally tubular body at its proximal portion. The inner tubular member 120 has a side-facing cutting member at its distal portion and a generally tubular proximal portion defining a sample storage space 126. The cutting member includes a side-facing opening 131 defined at its periphery by sharp cutting edges 122 and a distal end 123 that extends partially across the radius of the member 120. The member 120 is actuatable in two motions. It can be rotated about the device axis A to cut a sample from a tissue surface. It can also be moved axially to pull the cut sample into the storage space 126 where previous samples can be stored while subsequent samples are being taken, thus allowing multiple samples to be taken without removing the device from the endoscope.

Referring particularly to Fig. 2b, the interior walls of member 120 are provided with a low friction surface, for example, by providing a coating 132.

Referring as well to Fig. 2c, the sleeve 124 includes a roughened high friction segment 133 along its inner surface at a location adjacent the opening 129. The high friction segment 133 is provided at only a small axial and circumferential portion of the interior wall of the sleeve 124 so it does not interfere with the motions of the member the sleeve, in cooperation with the low friction coating 132 on the interior of the member 120, permits cut samples to be urged into the sample storage space 126 as the member 120 is advanced distally to prepare it for taking further samples. Moreover, the location of the segment and the construction and rotational actuation of the member 120 prevents the sample from being abraded by the segment 133 as it is drawn axially proximally. The member 120 is also provided with a scraping element 141 that extends radially somewhat so that it lightly contacts the interior surface of the sleeve to help guide the sample into the storage space.

The device also includes an axially moveable discharge pusher 125 for removing the samples from the storage space. A flexible coil 127 is attached to the proximal portion of the sleeve 124. The coil may be provided within a slippery, flexible polymer sleeve (not shown) as known in the art. The rotational and axial motions of the member 120 can be controlled from the proximal portions of the device outside the body by a torqueable control wire (not shown) that is attached to the distal portions of the member 120 and extends proximally.

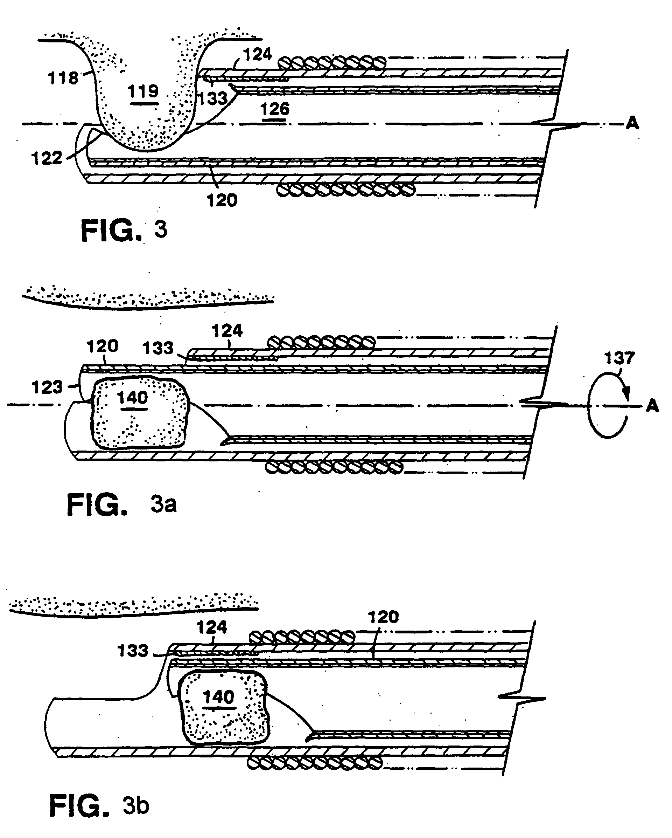

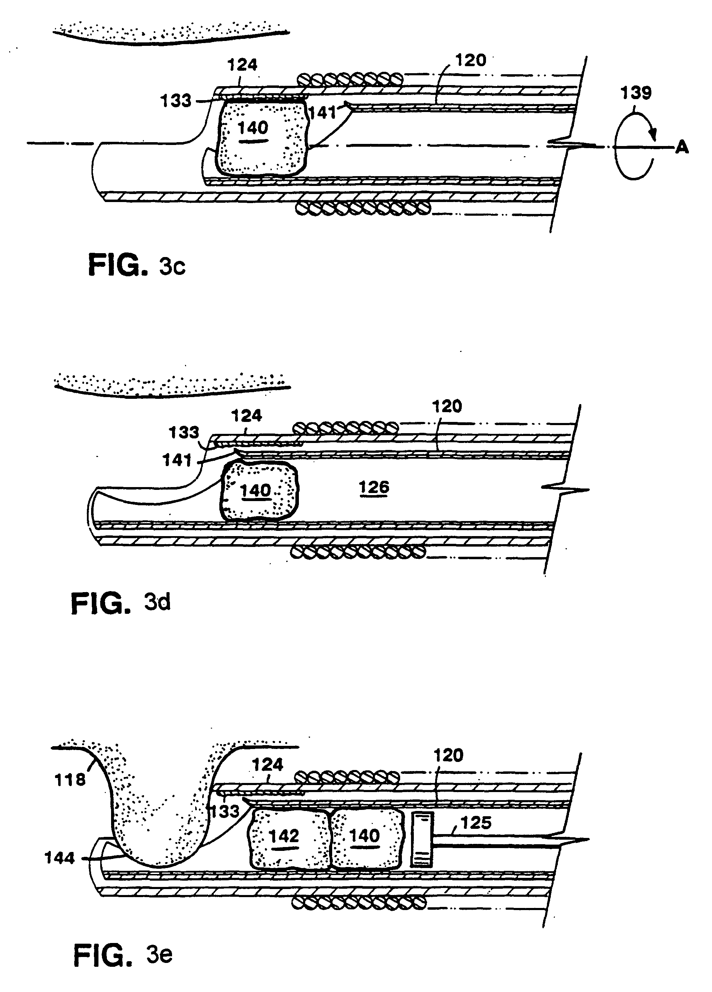

Referring to Figs. 3-3e, particularly to Fig. 3, in use, the device is positioned adjacent a tissue area, such as a polyp feature 119 which is to be sampled. The device may be urged against the tissue surface so that the feature 119 passes through the openings 129, 131 of the sleeve 124 and the member 120.

Referring particularly to Fig. 3a, the member 120 is rotated 180° about axis A causing a shearing action of cutting surface 122 upon the tissue surface 115 (arrow 137). The first sample, sample 140, is thus separated from the tissue surface and captured between the cutting portion of the member 120 and the interior walls of the sleeve 124.

Referring to Fig. 3b, the member 120 is then moved axially proximally, causing the radially extending end 123 of the member to engage the distal portion of the cut sample 140 and move the sample proximally. During axial motion, the sample is not rubbed against the high friction segment 133 of the sleeve. Instead, with the member 120 oriented as shown, the outer surface of the member 120 is exposed to the high friction segment and the sample 140 engages only the relatively low friction portions of the interior surface of the sleeve. This is an advantage since the sample is not abraded as it moves distally; abrading the sample could damage it and make later biopsy analysis more difficult. The sample is withdrawn proximally until it reaches an axial location that is in alignment with the high friction surface 133. Referring to Fig. 3c, the member 120 is again rotated 1500 about its axis. Because of the low friction surfaces on the inner walls of member 120, the member rotates about the sample, which remains substantially axially stationary, although it moves radially somewhat as the member 120 slips around it. By this rotation, the sample 140, extending through the opening 129, is placed in contact with the high friction segment 133 of the sleeve 124.

Referring to Fig. 3d, the member 120 is then extended distally to prepare the device for taking another sample. The sample 140 does not travel axially with the member 120 but is instead restrained from axial motion by the segment 133. Moreover, as the member moves axially, the sample slips into storage area 126 of member 120 since it slides easily on the high friction surfaces on the walls of member 120. The scraper 141, which is angled slightly so it engages the segment 133, acts like a spatula to help slip the sample into the storage area 15 as the member 120 moves distally.

Referring to Fig. 3e, subsequent samples 142 can be taken without removing the device from the endoscope by repeating the above sequence. Samples are brought into the sample storage space 126 in the order in which they were collected. To remove the samples from the device, the push rod 125 is moved axially distally and each sample is removed through the openings 129 and 131, for example, using forceps.

The member 120 and sleeve 124 can be formed of biocompatible materials such as stainless steel, aluminum, or plastics. The length of the member and the sleeve are relatively short, e.g., on the order of about 25mm (1 inch), so the device can be passed through tortuous passageways without being hung up because of the stiffness of the end 116. The device is preferably dimensioned to take and store at least five samples before removing it from the body. The high friction segment 133 may be formed, for example, by sandblasting or etching, or it may be formed by depositing a high friction adhesive coating on the inner wall of the sleeve. The low friction surface on the interior walls of the member 120 may be provided by depositing a coating of a polymer such as Teflon® or, more preferably, a hydrogel. A suitable hydrogel is discussed in Fan U.S. 5,091,205. For embodiments constructed for use in vascular applications, the hydrogel coating can be made antithrombogenic, as discussed in Sahatjian U.S. 5,135,516. A low friction coating may also be provided on the outer surfaces of the member 120 and the inner surfaces of the sleeve 124 (except for the segment 133) to reduce frictional resistance to the motions of the member 120. The low friction coating is low compared to the high friction segment 133 of the sleeve 124. In embodiments, the interior walls of the member 120 may have sufficiently low friction without a coating or further treatment. The scraper 14 may also have a high friction inner and/or outer surface. The scraper 141 preferably is a thin extension from the member 120 that has some elasticity that allows it to engage the high friction segment of the sleeve without binding as the member is moved distally. The scraper may be omitted in embodiments where there is sufficiently small clearance, e.g., on the order of 0.03mm (0.001 inch), between the sleeve 124 and the member 120. The proximal portion of the control wire outside the body may include index markings that indicate the axial location of the member 120 relative to the sleeve 124.

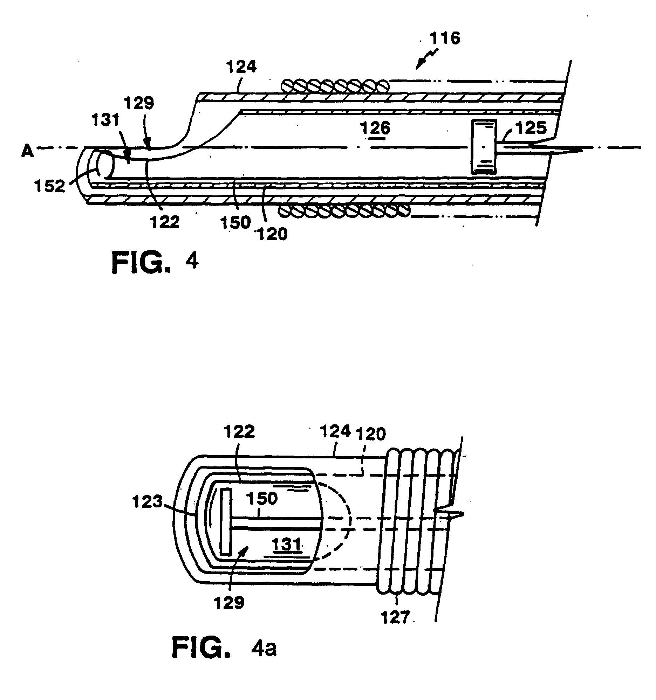

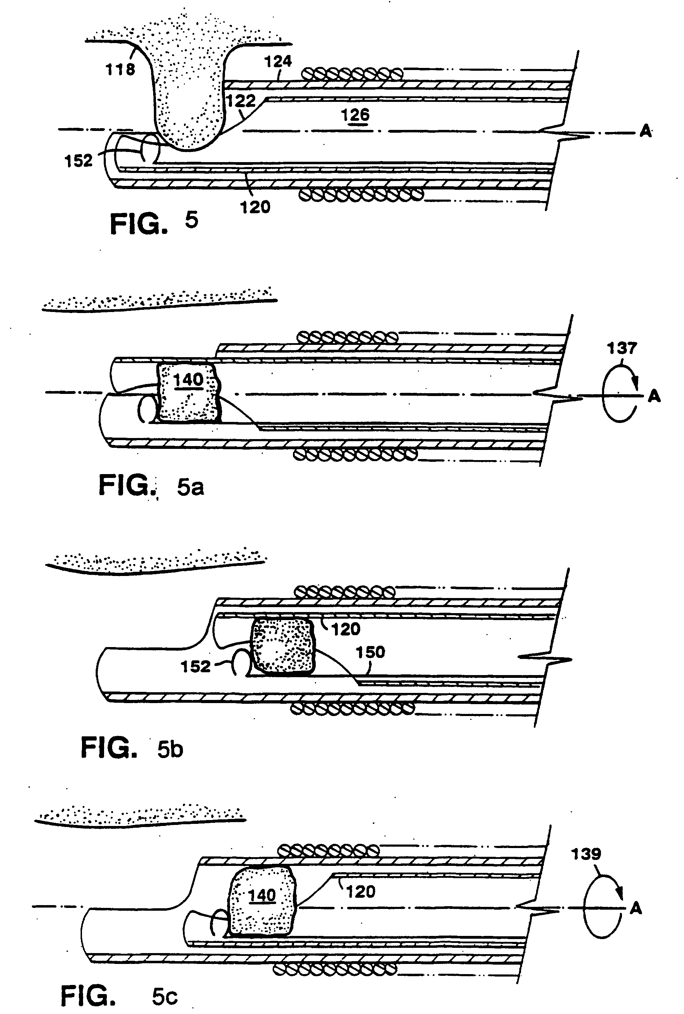

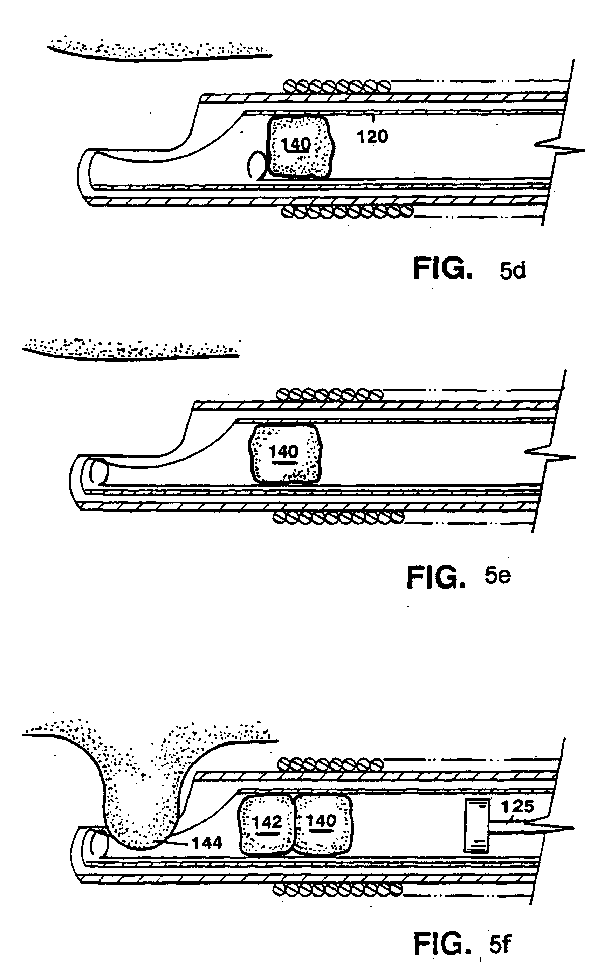

Referring to Figs. 4-4a and 5-5e, in another embodiment, the sampling portion 116 includes an axially moveable sample indexer 150 with a sample engaging head 152. In this embodiment, the high friction segment of the sleeve 124 may be omitted. Samples move proximally when engaged by the head but because the indexer 150 is narrow and may include a low friction coating samples do not move distally when the head is advanced (Fig. 4a).

Referring to Figs. 5-5f, particularly Fig. 5, in use, cutting surface 122 is first brought close to tissue surface 118 where a sample is to be taken. The sample indexer is extended axially such that the head 152 does not substantially obstruct the opening 129. Referring particularly to Fig. 5a, member 120 is rotated about axis A causing a shearing action of cutting surface 122 upon tissue surface 118. The sample is separated from the tissue and first sample 140 is collected.

Referring to Fig. 5b, both member 120 and sample indexer 150 are pulled axially proximally. The head 152 and the distal end 123 of the member 120 engage the sample and pull it axially proximally. Referring to Fig. 5c, the member 120 is then again rotated about axis A.

Referring to Fig. 5d, the member 120 is then pushed axially distally to prepare to take another sample. While the member 120 is being pushed axially distally, sample indexer 150 remains in its proximal position to retain sample 140 in sample storage space 126.

Referring to Fig. 5e, the sample indexer is then advanced axially distally to its original position. Subsequent samples can then be taken.

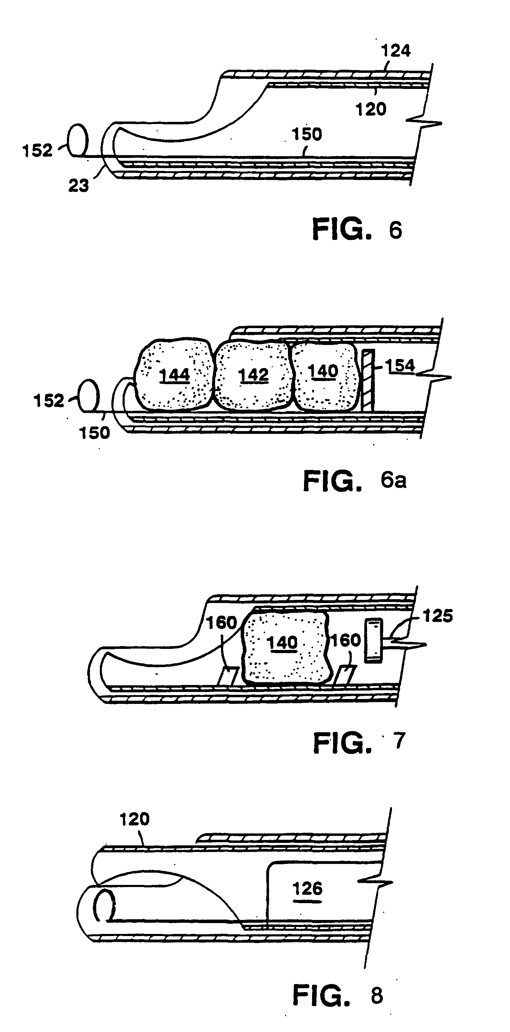

Referring to Fig. 5f, subsequent samples 142 and 144 can be taken without removing the device from the endoscope by repeating the above sequence. The samples are brought into the sample storage space in the order in which they were collected. To remove samples from the device, push rod 125 is moved axially distally and each sample is removed through access hole 128, for example, using forceps. Referring to Fig. 6, in other embodiments, member 120 and sleeve 124 are modified by placing a hole through their distal ends so that sample indexer 150 can thus be advanced to push the head 152 distally through the end 123 of the member 120 and sleeve 124 allowing more room for sample capturing and for sample access during sample removal. Referring to Fig. 6a, in embodiments, sample indexer 150 also carries a sample discharge surface 154 that can be used to remove samples from the storage space by extending the indexer distally.

Referring to Fig. 7, in embodiments, member 120 has index points 160 in the storage space. The index points 160 are flexible radial extensions from the inner wall of the member 120 that are angled to be easily bent when samples are urged proximally but inhibit the samples from moving distally. The index points 160 can be bent (elastically in a multi-use device) distally to remove the samples from the storage space when greater force is applied by the discharge pusher 125.

Referring to Fig. 8, in still further embodiments, proximal portions of member 120 are removed to reduce the frictional contact with the inner surface of the sleeve 124. The sample space 126 is defined by the remaining arc-form of the member 120 and the proximal portions of the sleeve 124.

Still other embodiments are within the following claims. For example, a barbed spear-form can also be used to hold samples in the storage area.

## Claims

1. An instrument (10) for obtaining tissue samples (140) from sites deep within a body (118), the instrument (10) having an elongate flexible proximal portion (127) that is constructed to follow a long, tortuous path to said sites, and having, at its distal end, a side-facing sampling assembly (116) constructed to remove a tissue sample (140) from the body (118), including tissue specimens, polyps or the like, wherein said instrument (10) is constructed to take multiple biopsy samples (140), without being withdrawn from the body (118), by means of a storage space (126) defined along the axis of said instrument (10) and proximal of the distal end of the instrument (10), suitable for storage of multiple, successively taken samples (140), and said side-facing sampling assembly (116) having a cutting member (120) near the distal end of said instrument (10) actuatable in a first, rotational motion about the axis of said instrument (10) to take a tissue sample (140) from the body and in a second, limited axial motion towards the proximal end of the instrument to take said sample between a sample cutting position and a position in said storage space (126) for disposing said sample (140) axially into said storage space (126), said cutting member (120) in use being returnable to the sample cutting position in preparation for taking a successive sample (140), the previous sample or samples being retained axially in said storage space, without withdrawing said cutting member (120) or said instrument (10) from the body (118).

2. The instrument (10) of claim 1, wherein said sampling assembly (116) has an integral, axially adjacent sample-engaging surface (123) for engaging a sample (140) during said axial motion of said element.

3. The instrument (10) of claim 2, wherein said sampling assembly (116) is a tube-form including a side-facing cutout (131) defining a cutting surface (122) and said sample engaging surface (123), and said storage space (126) is defined by a portion of said tube-form adjacent said cutout.

4. The instrument (10) of claim 1, including a retention formation (133) constructed such that said sample (140) can be moved in the proximal direction into contact with said retention formation by the said second limited axial motion of the cutting member and said cutting member (120) can be returned to the sample cutting position while said sample (140) is retained axially stationary by said retention formation.

5. The instrument (10) of claim 4, wherein said retention formation (133) is a high friction surface.

6. The instrument (10) of claim 5, wherein said sampling assembly (116) is constructed to prevent abrasion of said sample (140) against said retention formation (133) as said sample (140) is moved axially in the proximal direction.

7. The instrument (10) of claim 6, including an outer sleeve (124) wherein said cutting member (120) is movable relative to said outer sleeve, and said retention formation (133) is provided on said outer sleeve.

8. The instrument (10) of claim 7, wherein said retention formation (133) is provided on a small portion of the interior surface of said outer sleeve (124) that is about equal to or less than the circumferential width and axial length of the side-facing cutout (131) of said sampling assembly.

9. The instrument (10) of claim 8, wherein said sampling assembly includes a scraping surface (141) constructed to move between a tissue sample (140) and said retention formation (133) to facilitate transfer of said sample (140) into said storage space (126) as said cutting member (120) returns to the sample cutting position.

10. The instrument (10) of claim 3 and claim 9, wherein said outer sleeve (124) includes a side-facing opening (129) through which tissue may pass to be engaged by said cutting surface of said cutting member (120).

11. The instrument (10) of claim 4, wherein said retention formation (133) is formed by sandblasting.

12. The instrument (10) of claim 1, wherein interior sample-contacting surfaces of said storage space (126) include a low friction coating (132) that allows easy axial relative displacement of the surfaces and said sample (140).

13. The instrument (10) of claim 12, wherein said low friction coating is a hydrogel.

14. The instrument (10) of claim 1, further including an axially movable sample-retaining element (152) constructed to engage and maintain samples (140) in said storage space (126) as said cutting member (120) moves axially into a cutting position.

## Patentansprüche

1. Ein Instrument (10), um Gewebeproben (140) von Stellen tief innerhalb eines Körpers (118) zu erhalten, wobei das Instrument (10) einen verlängerten flexiblen proximalen Teil (127), konstruiert um einem langen, gewundenen Pfad zu den genannten Stellen zu folgen, und, an seinem distalen Ende, eine zur Seite gewandte Proben-Entnahmeanordnung (116), konstruiert, um eine Gewebeprobe (140) , einschließlich Gewebeteilen, Polypen oder ähnlichem, aus dem Körper (118) zu entnehmen, besitzt, wobei das Instrument (10) konstruiert ist, um mehrere Biopsie-Proben (140) zu entnehmen, ohne aus dem Körper (118) zurückgezogen zu werden, mittels eines Aufnahmeraums (126), welcher entlang der Achse des Instruments und proximal zum distalen Ende des Instruments (10) gebildet wird und für die Aufnahme mehrerer, hinter einander entnommener Proben (140) geeignet ist, und die zur Seite gewandte Proben-Entnahmeanordnung (116) ein Schneideteil (120) proximal zum distalen Ende des Instruments besitzt, welches in einer ersten Drehung um die Achse des Instruments (10), um eine Gewebeprobe (140) aus dem Körper zu entnehmen und in einer zweiten, begrenzten axialen Bewegung gegen das proximale Ende des Instruments bewegt werden kann, um die genannte Probe zwischen eine Proben-Schneideposition und eine Position in dem Aufnahmeraum (126) zu bewegen, um die Probe (140) axial in dem Aufnahmeraum (126) abzulegen, wobei das benutzte Schneideteil (120) an die Proben-Schneideposition zurückführbar ist, in Vorbereitung der Entnahme einer folgenden Probe (140), wobei die vorhergehende Probe axial in dem Aufnahmeraum zurückbehalten wird, ohne das Schneideteil (120) oder das Instrument (10) aus dem Körper (118) zurückzuziehen.

2. Instrument (10) gemäß Anspruch 1, wobei die Proben-Entnahmeanordnung (116) als integralen Bestandteil axial benachbarte Proben-Aufnahmefläche (123), zur Aufnahme einer Probe (140) während der Bewegung des Elements entlang der Achse, besitzt.

3. Instrument (10) gemäß Anspruch 2, wobei die Proben-Entnahmeanordnung (116) schlauchförmig ist, einschließlich eines zur Seite gewandten Ausschnitts (131), welcher eine Schneidefläche (122) bildet, sowie der Proben-Aufnahmefläche (123), und der Aufriahmeraum (126) gebildet wird durch einen Abschnitt der Schlauchform, welcher dem Ausschnitt benachbart ist.

4. Instrument (10) gemäß Anspruch 1, einschließlich einer Rückhalte-Anordnung (133), so konstruiert, dass die Probe (140) durch Bewegung in proximaler Richtung mit der Rückhalte-Anordnung in Kontakt gebracht werden kann, durch die zweite begrenzte axiale Bewegung des Schneideteils und das Schneideteil (120) an die Proben-Schneideposition zurückgeführt werden kann, während die Probe (140) durch die Rückhalte-Anordnung axial festgehalten wird.

5. Instrument (10) gemäß Anspruch 4, wobei die Rückhalte-Anordnung (133) eine Oberfläche mit hoher Reibung ist.

6. Instrument (10) gemäß Anspruch 5, wobei die Proben-Entnahmeanordnung (116) konstruiert ist, um ein Abreiben der Probe (140) gegen die Rückhalte-Anordnung (133) zu verhindern, während die Probe (140) axial in proximaler Richtung bewegt wird.

7. Instrument (10) gemäß Anspruch 6, einschließlich einer äußeren Hülse (124), wobei das Schneideteil (120) relativ zu der äußeren Hülse bewegt werden kann und die Rückhalte-Anordnung (133) auf der äußeren Hülse vorgesehen ist.

8. Instrument (10) gemäß Anspruch 7, wobei die Rückhalte-Anordnung (133) auf einem kleinen Abschnitt der inneren Oberfläche der äußeren Hülse (124) bereitgestellt wird, der ungefähr gleich groß oder kleiner als die Umfangsbreite und axiale Länge des zur Seite gewandten Ausschnitts (131) der Proben-Entnahmeanordnung ist.

9. Instrument (10) gemäß Anspruch 8, wobei die Proben-Entnahmeanordnung eine Schabe-Fläche (141) umfaßt, so aufgebaut, dass sie sich zwischen einer Gewebeprobe (140) und der Rückhalte-Anordnung (133) bewegt, um den Transfer der Probe (140) in den Aufnahmeraum (126) zu erleichtern, während das Schneideteil (120) zur Proben-Schneideposition zurückkehrt.

10. Instrument (10) gemäß Anspruch 3 und/oder Anspruch 9, wobei die äußere Hülse (124) eine zur Seite gewandte Öffnung (129) umfaßt, durch welche Gewebe hindurch treten kann, um von der Schneidefläche des Schneideteils (120) aufgenommen zu werden.

11. Instrument (10) gemäß Anspruch 4, wobei die Rückhalte-Anordnung (133) durch Sandstrahlen gebildet wird.

12. Instrument (10) gemäß Anspruch 1, wobei innere Proben-berührende Oberflächen des Aufhahmeraums (126) eine Beschichtung mit niedriger Reibung (132) beinhalten, welche eine axiale Verschiebung von Oberflächen und der Probe (140) relativ zueinander erleichtert.

13. Instrument (10) gemäß Anspruch 12, wobei die Beschichtung mit niedriger Reibung ein Hydrogel ist.

14. Instrument (10) gemäß Anspruch 1, ferner ein axial bewegbares Proben-Rückhalteelement (152) beinhaltend, aufgebaut um Proben (140) aufzunehmen und in dem Aufnahmeraum (126) zu behalten, während das Schneideteil (120) sich axial in die Schneideposition bewegt.

## Revendications

1. Un instrument (10) pour obtenir des échantillons de tissus (140) à partir de sites profonds à l'intérieur d'un corps (118), l'instrument (10) ayant une partie proximale souple allongée (127) qui est construite pour suivre un chemin long, tortueux vers lesdits sites et ayant, à son extrémité distale, un ensemble d'échantillonnage donnant sur le côté (116) construit pour retirer un échantillon de tissu (140) du corps (118), y compris des prélèvements de tissus, polype ou l'équivalent, dans lequel ledit instrument (10) est construit pour prélever de multiples échantillons biopsiques (140) sans être retiré du corps (118) grâce à un espace de stockage (126) défini le long de l'axe dudit instrument (10) et proche de l'extrémité distale de l'instrument (10), adapté pour le stockage de multiples échantillons prélevés successivement (140), et ledit ensemble d'échantillonnage donnant sur le côté (116) ayant un organe de coupe (120) proche de l'extrémité distale dudit instrument (10) pouvant être actionné dans un premier mouvement de rotation autour de l'axe dudit instrument (10) pour prélever un échantillon de tissu (140) dans le corps, et actionné dans un second mouvement axial limité en direction de l'extrémité proximale de l'instrument pour déplacer ledit échantillon entre une position de coupe de l'échantillon et une position dans ledit espace de stockage (126) afin de disposer ledit échantillon (140) axialement dans ledit espace de stockage (126), ledit organe de coupe (120), en utilisation, pouvant être renvoyé à la position de coupe d'échantillon en préparation du prélèvement d'un échantillon suivant (140), l'échantillon ou les échantillons précédents étant retenus axialement dans ledit espace de stockage, sans retirer ledit organe de coupe (120) ou ledit instrument (10) du corps (118).

2. L'instrument (10) de la revendication 1, dans lequel ledit ensemble d'échantillonnage (116) a une surface intégrée de prise de l'échantillon (123), adjacente axialement, pour prendre un échantillon (140) pendant ledit mouvement axial dudit élément.

3. L'instrument (10) de la revendication 2, dans lequel ledit ensemble d'échantillonnage (116) est une forme de tube incluant une découpe (131) donnant sur le côté définissant une surface de coupe (122) et ladite surface de prise de l'échantillon (123), et ledit espace de stockage (126) est défini par une portion de ladite forme de tube adjacente à ladite découpe.

4. L'instrument (10) de la revendication 1, comprenant une structure de rétention (133) construite de telle façon que ledit échantillon (140) puisse être déplacé dans la direction proximale jusqu'au contact de ladite structure de rétention par ledit second mouvement axial limité de l'organe de coupe et ledit organe de coupe (120) peut être renvoyé à la position de coupe d'échantillon tandis que ledit échantillon (140) est retenu axialement fixe par ladite structure de rétention.

5. L'instrument (10) de la revendication 4, dans lequel ladite structure de rétention (133) est une surface à frottement élevé.

6. L'instrument (10) de la revendication 5, dans lequel ledit ensemble d'échantillonnage (116) est construit pour empêcher l'abrasion dudit échantillon (140) contre ladite structure de rétention (133) lorsque ledit échantillon (140) est déplacé axialement dans la direction proximale.

7. L'instrument (10) de la revendication 6, incluant un manchon extérieur (124) dans lequel ledit organe de coupe (120) est mobile par rapport audit manchon extérieur, et ladite structure de rétention (133) est fournie sur ledit manchon extérieur.

8. L'instrument (10) de la revendication 7, dans lequel ladite structure de rétention (133) est fournie sur une petite partie de la surface intérieure dudit manchon extérieur (124) qui est approximativement égale ou inférieure à la largeur circonférentielle et à la longueur axiale de la découpe donnant sur le côté (131) dudit ensemble d'échantillonnage.

9. L'instrument (10) de la revendication 8, dans lequel ledit ensemble d'échantillonnage inclut une surface racleuse (141) construite pour se déplacer entre un échantillon de tissu (140) et ladite structure de rétention (133) afin de faciliter le transfert dudit échantillon (140) dans ledit espace de stockage (126) lorsque ledit organe de coupe (120) revient à la position de découpe d'échantillon.

10. L'instrument (10) de la revendication 3 et de la revendication 9, dans lequel ledit manchon extérieur (124) inclut une ouverture donnant sur le côté (129) par laquelle du tissu peut passer pour être pris par ladite surface de coupe dudit organe de coupe (120).

11. L'instrument (10) de la revendication 4, dans lequel ladite structure de rétention (133) est formée par sablage.

12. L'instrument (10) de la revendication 1, dans lequel les surfaces intérieures au contact de l'échantillon dudit espace de stockage (126) comprennent un revêtement à frottement réduit (132) qui permet un déplacement relatif axial facile des surfaces et dudit échantillon (140).

13. L'instrument (10) de la revendication 12, dans lequel ledit revêtement à frottement réduit est un hydrogel.

14. L'instrument (10) de la revendication 1, comprenant en outre un élément mobile axialement de retenue de l'échantillon (152) construit pour prendre et maintenir des échantillons (140) dans ledit espace de stockage (126) lorsque ledit organe de coupe (120) se déplace axialement jusqu'à une position de coupe.
